(19) Europäisches Patentamt
European Patent Office
Office européen des brevets

(11) Veröffentlichungsnummer: **0 063 678**
**B1**

(12) # EUROPÄISCHE PATENTSCHRIFT

(45) Veröffentlichungstag der Patentschrift: **21.08.85**

(21) Anmeldenummer: **82101198.8**

(22) Anmeldetag: **18.02.82**

(51) Int. Cl.⁴: **C 07 J 9/00**, C 07 J 17/00, C 07 J 31/00, C 07 J 53/00, C 07 D 317/26

(54) **Verfahren zur Herstellung von Cholesterinderivaten und Zwischenprodukte dafür.**

(30) Priorität: **29.04.81 CH 2784/81**

(43) Veröffentlichungstag der Anmeldung: **03.11.82 Patentblatt 82/44**

(45) Bekanntmachung des Hinweises auf die Patenterteilung: **21.08.85 Patentblatt 85/34**

(84) Benannte Vertragsstaaten: **AT BE CH DE FR GB IT LI NL**

(56) Entgegenhaltungen:
**EP - A - 0 045 524**
**FR - A - 2 343 727**
**US - A - 3 994 878**

**TETRAHEDRON LETTERS, Band 21, Nr. 52, 1980, Seiten 5027-5028 Pergamon Press OXFORD (GB) HIROAKI TAKAYAMA et al.: "Facile stereoselective synthesis of (24R)-24,25- dihydroxyvitamin D3 using D-glyceric acid as a chiral synthon"**

(73) Patentinhaber: **F. HOFFMANN-LA ROCHE & CO. Aktiengesellschaft, CH-4002 Basel (CH)**

(72) Erfinder: **Fürst, Andor, Dr., Magnolienpark 14, CH-4052 Basel (CH)**
Erfinder: **Labler, Ludwig, Dr., Ochsengasse 10, CH-4123 Allschwil (CH)**
Erfinder: **Meier, Werner, Dr., Bierastrasse 5, CH-4103 Bottmingen (CH)**

(74) Vertreter: **Mahé, Jean et al, Postfach 3255 Grenzacherstrasse 124, CH-4002 Basel (CH)**

EP 0 063 678 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die Erfindung betrifft ein neues Verfahren zur Herstellung von Cholesterinderivaten der Formel

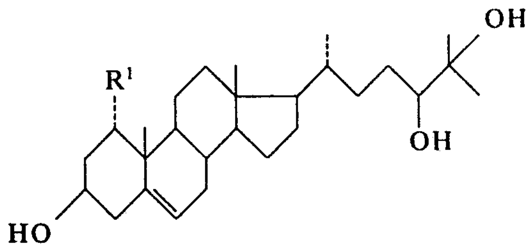

(I)

worin $R^1$ Wasserstoff oder Hydroxy ist,
und neue Zwischenprodukte dafür.

Dieses Verfahren ist dadurch gekennzeichnet, daß man

a) ein Pregnanderivat der Formel

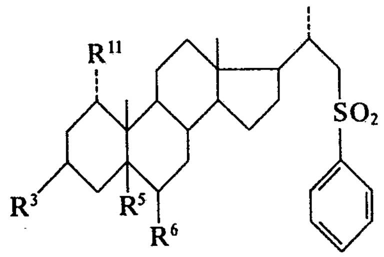

(II)

worin $R^{11}$ Wasserstoff, $R^3$ und $R^5$ zusammen eine $3\alpha$,5-Bindung und $R^6$ $C_{1-4}$-Alkoxy sind, oder $R^{11}$ Wasserstoff oder eine leicht spaltbare verätherte Hydroxygruppe, $R^3$ eine leicht spaltbare verätherte Hydroxygruppe und $R^5$ und $R^6$ zusammen eine C—C-Bindung sind,
mit einer Verbindung der Formel

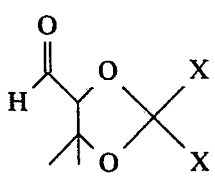

(III)

worin X Wasserstoff oder Methyl ist,
umsetzt,

b) aus der erhaltenen Verbindung der Formel

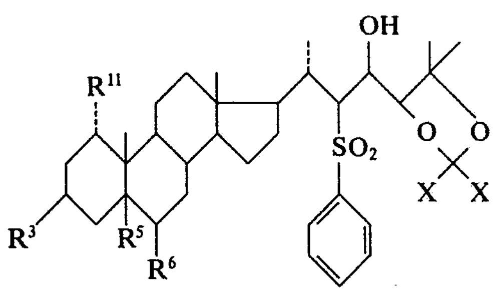

(IV)

worin X, $R^{11}$, $R^3$, $R^5$ und $R^6$ die obige Bedeutung haben,
die $\beta$-Hydroxysulfongruppierung reduktiv eliminiert,

c) die erhaltene Verbindung der Formel

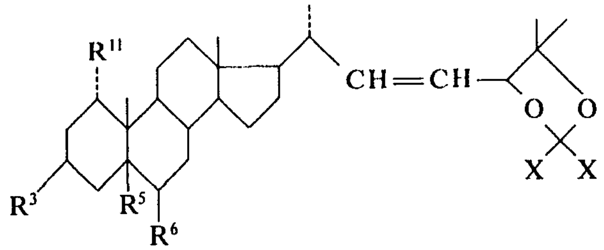

(V)

worin X, $R^{11}$, $R^3$, $R^5$ und $R^6$ die obige Bedeutung haben, katalytisch hydriert,

d) die erhaltene Verbindung der Formel

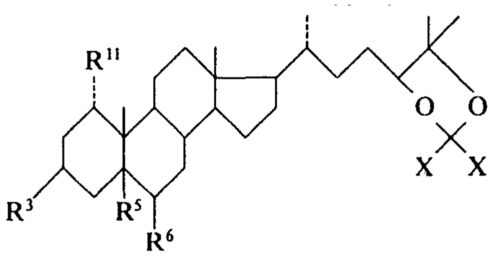

(VI)

worin X, $R^{11}$, $R^3$, $R^5$ und $R^6$ die obige Bedeutung haben, zu einer Verbindung der Formel I hydrolysiert und

e) erwünschtenfalls ein erhaltenes Diastereomerengemisch von Verbindungen der Formel I auftrennt.

Die Verbindungen der Formel I sind bekannte Zwischenprodukte in der Herstellung der Vitamin-$D_3$-Derivaten 24,25-Dihydroxy- und 1α,24,24-Trihydroxycholecalciferol und können in diese in bekannter Weise (vgl. DE-OS 2 710 062) oder in Analogie dazu übergeführt werden.

Beispiele von $C_{1-4}$-Alkoxyresten sind Methoxy, Äthoxy, n-Propoxy, Isopropoxy und Butyloxy, vorzugsweise Methoxy. Äthergruppen $R^{11}$ oder $R^3$, die sich leicht, d. h. ohne Veränderung an anderen Stellen des Moleküls spalten lassen, sind vorzugsweise solche der Formel

$$R'O-C(R,R'')-O-,$$

worin R Wasserstoff oder $C_{1-4}$-Alkyl, R' und R" $C_{1-4}$-Alkyl oder R' und R" gemeinsam $C_{3-6}$-Alkylen sind. Besonders bevorzugte Äthergruppen sind Tetrahydro-2H-pyran-2-yloxy und 1-Äthoxyäthoxy.

Zur Herstellung der 24R-Diastereomeren der Formel I wird vorzugsweise eine Verbindung der Formel III, worin X Methyl ist, insbesondere (S)-2,2,5,5-Tetramethyl-1,3-dioxolan-4-aldehyd, als Ausgangsmaterial verwendet. Man kann jedoch auch von (RS)-2,2,5,5-Tetramethyl-1,3-dioxolan-4-aldehyd ausgehen und das erhaltene 24RS-Gemisch der Formel I in die Diastereomeren auftrennen.

Zur Umsetzung einer Verbindung der Formel II mit einer solchen der Formel III wird zweckmäßig die Verbindung der Formel II zunächst mit einem $C_{1-4}$-Alkylmagnesiumhalogenid, wie Methylmagnesiumbromid, oder mit einem $C_{1-4}$-Alkyllithium, wie Butyllithium, in einem inerten Lösungsmittel, wie einem Kohlenwasserstoff, z. B. Benzol oder Toluol,oder einem Äther, z. B. Tetrahydrofuran, zum Magnesium- bzw. Lithiumsalz umgesetzt. Die Herstellung des Magnesiumsalzes erfolgt zweckmäßig unter Erhitzen, vorzugsweise durch Kochen unter Rückfluß, diejenige des Lithiumsalzes zweckmäßig bei Temperaturen unter $-20°C$, vorzugsweise bei $-75°C$. Die Temperatur, bei der die anschließende Kondensation mit dem Aldehyd der Formel III durchgeführt wird, ist bei Verwendung des Magnesiumsalzes nicht kritisch; sie wird vorzugsweise bei Raumtemperatur durchgeführt. Im Falle des Lithiumsalzes wird sie zweckmäßig bei $-20°C$ bis Raumtemperatur durchgeführt.

Die Verfahrensstufe b) wird mit dem Additionsprodukt der Formel IV durchgeführt werden. Die Ausbeute an (22)-Olefin der Formel V ist jedoch höher, wenn man zunächst das Additionsprodukt in ein n-$C_{1-4}$-Alkanoylat, wie das Formiat, Acetat, n-Propionat oder n-Butyrat, oder ein Sulfonat, wie das Mesylat, oder ein Thiolat, wie das Thioacetat, umwandelt und dann die β-Acyloxysulfongruppierung aus dem erhaltenen Ester reduktiv eliminiert. Da die C(23)-Hydroxygruppe von gewissen Isomeren der Formel IV sterisch gehindert ist, sind je nach Acylierungsmittel energischere Reaktionsbedingungen notwendig. Für die Acetylierung z. B. ist eine Behandlung mit Acetanhydrid in Triäthylamin oder Pyridin in Gegenwart von 4-Dimethylaminopyridin, zweckmäßig unter Rückfluß, angezeigt. Die Reduktion des entstandenen Acylats gelingt mit einem Alkalimetallamalgam, wie dem Natriumamalgam, in

einem Lösungsmittel, wie einem $C_{1-4}$-Alkanol, z. B. Methanol, gegebenenfalls in Gegenwart von einem Äther, z. B. Tetrahydrofuran, oder von Äthylacetat als Cosolvens, bei einer Temperatur bis zu $-10°$ C, vorzugsweise bei Raumtemperatur.

Die katalytische Hydrierung der Verbindungen der Formel V kann mit Raney-Nickel oder mit Platin oder Palladium auf Kohle in einem Lösungsmittel, wie einem $C_{1-4}$-Alkanol, z. B. Äthanol, oder einem Äther, z. B. Tetrahydrofuran, durchgeführt werden. Durch Zusatz einer Base, wie einem Alkalimetallbicarbonat, z. B. Natriumbicarbonat, oder eines tert. Amins, wie Triäthylamin, wird das Hydrierungsmedium schwach alkalisch bis neutral gehalten. Die Temperatur und der Druck sind nicht kritisch. Vorzugsweise arbeitet man jedoch bei Raumtemperatur und Normaldruck.

Die Hydrolyse einer Verbindung der Formel VI zu einer solchen der Formel I kann durch Behandlung mit einer Säure in einem solvolytischen Medium bewerkstelligt werden. Als solvolytisches Medium kommen wäßrige Medien in Betracht, die ein mischbares Cosolvens enthalten. Geeignete Cosolventien sind ätherische Lösungsmittel, wie Dioxan oder Tetrahydrofuran, Ketone, wie Aceton und Methyläthylketon, oder Alkohole, wie Äthanol. Als Säuren kommen Mineralsäuren, wie Salzsäure, Bromwasserstoffsäure oder Schwefelsäure, oder organische Sulfonsäuren, wie p-Toluolsulfonsäure oder Benzolsulfonsäure, in Betracht. Die Hydrolyse wird zweckmäßig bei erhöhter Temperatur, z. B. bei etwa 40—120° C, vorzugsweise bei etwa 80° C, durchgeführt.

Die Auftrennung eines so erhaltenen Diastereomerengenmisches von Verbindungen der Formel I kann zweckmäßig über Esterderivate, z. B. das Tri- bzw. Tetraacetat, mittels chromatographischen Methoden erfolgen. Die vollständige Acetylierung einer Verbindung der Formel I kann in Analogie zu der weiter oben für die Verbindungen der Formel IV beschriebenen durchgeführt werden. Die Auftrennung der erwähnten Esterderivate kann in an sich bekannter Weise, z. B. durch Dünnschicht- und Säulenchromatographie, durchgeführt werden.

Die Verbindungen der Formeln IIc und V sind ebenfalls Gegenstand der vorliegenden Erfindung.

Die Sulfone der Formel II, worin $R^3$ und $R^5$ zusammen eine 3α,5-Bindung darstellen, d. h. Verbindungen der Formel

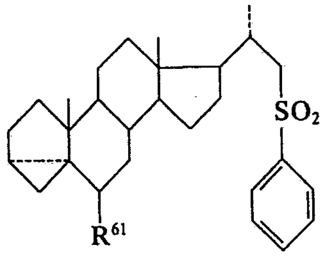

(IIa)

worin $R^{61}$ $C_{1-4}$-Alkoxy ist,
können durch Umsetzung der entsprechenden Jodide der Formel

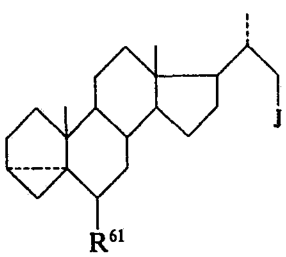

(VII)

worin $R^{61}$ die obige Bedeutung hat,
mit Natriumbenzolsulfinat in Dimethylformamid hergestellt werden.

Sulfone der Formel

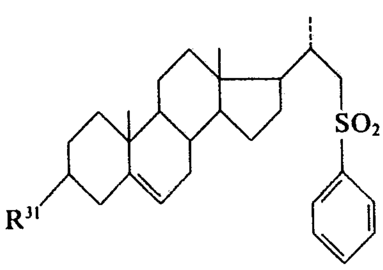

(IIb)

worin $R^{31}$ eine leicht spaltbare verätherte Hydroxygruppe ist,

können beispielsweise durch retro-i-Steroidumlagerung eines Sulfons der Formel IIa mit einer Säure, in Analogie zu der weiter oben beschriebenen Hydrolyse einer Verbindung der Formel VI zu einer solchen der Formel I, und anschließende Verätherung hergestellt werden.

Die Diäther der Formel II, d. h. die Verbindungen der Formel

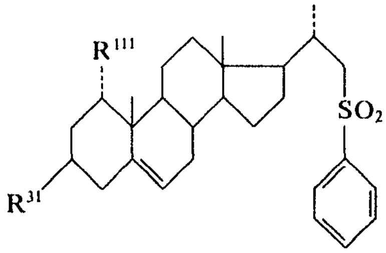

(IIc)

worin $R^{111}$ und $R^{31}$ leicht spaltbare verätherte Hydroxygruppen sind, können durch Erhitzen von (20S)-1α,3β-Diacetoxy-20-methyl-21-p-toluolsulfonyloxy-pregn-5-en mit Natriumjodid in Dimethylformamid, Umsetzung des erhaltenen (20S)-1α,3β-Diacetoxy-21-jod-20-methyl-pregn-5-ens mit Natriumbenzolsulfinat in Dimethylformamid, reduktive Deacetylierung des erhaltenen (20S)-1α,3β-Diacetoxy-20-methyl-21-phenylsulfonyloxy-pregn-5-ens, z. B. mit Lithiumaluminiumhydrid, und Verätherung des erhaltenen (20S)-1α,3β-Dihydroxy-20-methyl-21-phenylsulfonyl-pregn-5-ens hergestellt werden.

Die Verbindungen der Formel III in R- oder S-Form und als Racemat können in folgender Weise oder in Analogie dazu hergestellt werden:

(R)-2,2-Dimethyl-1,3-dioxolan-4-carbonsäure-methylester wird mit Methylmagnesiumbromid zu (R)-α,α,2,2-Tetramethyl-1,3-dioxolan-4-methanol umgesetzt, letzteres mit einer Säure, z. B. p-Toluolsulfonsäure, zum (R)-3-Methyl-1,2,3-butantriol hydrolysiert, dieses, z. B. mit Pivalinsäurechlorid in Pyridin, zum (R)-(2,3-Dihydroxy-3-methylbutyl)-pivalat umgesetzt, dieses mit Aceton zum (R)-(2,2,5,5-Tetramethyl-1,3-dioxolan-4-yl)-methylpivalat acetalisiert und letzteres, z. B. mit Alkali, zu (R)-2,2,5,5-Tetramethyl-1,3-dioxolan-4-methanol verseift. Diese Verbindung kann auch durch Umacetalisierung von (R)-α,α,2,2-Tetramethyl-1,3-dioxolan-4-methanol in Gegenwart von p-Toluolsulfonsäure hergestellt werden. Das erhaltene (R)-2,2,5,5-Tetramethyl-1,3-dioxolan-4-methanol kann dann z. B. mit Pyridiniumchlorochromat zu (S)-2,2,5,5-Tetramethyl-1,3-dioxolan-4-aldehyd oxydiert werden.

Ferner kann das in Anlehnung an das in Acta Chem. Scand. 23 (1969), 967, beschriebene Verfahren, aus β,β-Dimethylacrylsäure über (RS)-2,3-Dihydroxy-3-methylbuttersäure herstellbare (RS)-3-Methyl-1,2,3-butantriol mit Benzylbromid und einer Base in das (RS)-1-Benzyloxy-3-methyl-2,3-butandiol umgewandelt, dieses zu (RS)-5-Benzyloxymethyl-2,2,4,4-tetramethyl-1,3-dioxolan acetalisiert und dieses zum (RS)-2,2,5,5-Tetramethyl-1,3-dioxolan-4-methanol hydrogenolysiert werden. Letzteres kann auch in Analogie zu dem weiter oben in der (R)-Reihe beschriebenen Verfahren, aus (RS)-3-Methyl-1,2,3-butantriol über (RS)-(2,3-Dihydroxy-3-methylbutyl)-pivalat und (RS)-(2,2,5,5-Tetramethyl-1,4-dioxolan-4-yl)-methylpivalat hergestellt werden. Alternativ kann (RS)-2,2,5,5-Tetramethyl-1,3-dioxolan-4-methanol durch Umsetzung von (RS)-3-Methyl-1,2,3-butantriol mit Aceton und p-Toluolsulfonsäure zu einem durch Destillation trennbaren Gemisch von (RS)-α,α,2,2-Tetramethyl-1,3-dioxolan-4-methanol und (RS)-2,2,5,5-Tetramethyl-1,3-dioxolan-4-methanol hergestellt werden. Wie weiter oben in der (R)-Reihe beschrieben, kann dann das (RS)-2,2,5,5-Tetramethyl-1,3-dioxolan-4-methanol zum (RS)-2,2,5,5-Tetramethyl-1,3-dioxolan-4-aldehyd oxydiert werden.

Die FR-A-2 343 727 betrifft u. a. ein Verfahren zur Herstellung von unter die obige Formel VI fallenden Cholestanderivaten (der Formel XIV in der FR-A), ausgehend von anderen Cholestanderivaten (der Formel XV). Hingegen werden nach dem Verfahren der vorliegenden Erfindung Cholestanderivate durch Verlängerung der Seitenkette in Pregnanderivaten der Formel II hergestellt. Diese Verlängerung der Seitenkette wird durch Umsetzung mit Aldehyden der Formel III, hingegen in Tetrahedron Letters 21 (1980), 5027, mit Butantriolderivaten oder Epoxiden (der Formel 5 oder 6) durchgeführt.

Die nachfolgenden Beispiele erläutern die Erfindung.

## Beispiel 1

### a) (22RS,23RS,24R)-23-Hydroxy-24,25-isopropylidendioxy-6β,methoxy-22-phenylsulfonyl-3α,5-cyclo-5α-cholestan

Eine Lösung von 5,0 g (10,6 mMol) (20S)-6β-Methoxy-20-methyl-21-phenylsulfonyl-3α,5-cyclo-5α-pregnan in 95 ml Benzol wird mit 3,44 ml 3,4M ätherischer Methylmagnesiumbromid-Lösung (11,68 mMol) versetzt. Aus dem Gemisch wird bis zu einer Siedetemperatur von 77—78° Lösungsmittel abdestilliert und der Rückstand 7 Stunden unter Rückfluß erwärmt. Nach Abkühlung auf Raumtemperatur werden 2,016 g (12,7 mMol) (S)-2,2,5,45-Tetramethyl-1,3-dioxolan-4-aldehyd zugefügt. Nach 30

Minuten Rühren bei Raumtemperatur wird das Reaktionsgemisch in ein Gemisch von 100 ml gesättigter Ammoniumchlorid-Lösung und 100 g Eis gegossen und die organische Phase abgetrennt. Letztere ergibt nach Waschen mit gesättigter Kochsalz-Lösung, Trocknen über Natriumsulfat und Eindampfen bei 30°/1467 Pa 6,46 g farbloses Harz.

b) (22RS,23RS,24R)-23-Acetoxy-24,25-isopropylidendioxy-6β-methoxy-phenylsulfonyl-3α,5-cyclo-5α-cholestan

Eine Lösung des in a) erhaltenen Produktes (6,46 g) in 104 ml Triäthylamin wird mit 12,5 ml Acetanhydrid und 2,1 g 4-Dimethylaminopyridin versetzt und 3 Stunden unter Rückfluß erwärmt. Die abgekühlte Reaktionslösung wird in ein Gemisch von 200 ml gesättigter Natriumhydrogencarbonat-Lösung und 100 g Eis gegossen und mit Äther extrahiert. Der Extrakt wird mit gesättigter Kochsalz-Lösung gewaschen, über Natriumsulfat getrocknet und bei 30°/1467 Pa eingedampft. Der Rückstand wird in 400 ml Toluol gelöst und bei 40°/1467 Pa eingedampft. Das verbliebene braune Öl wird in Ätherlösung über eine Säule von 170 g Kieselgel filtriert und mit Äther nachgewaschen. Nach Eindampfen des Filtrats bei 30°/1467 Pa werden 6,46 g gelbes Harz erhalten.

c) (24R)-24,25-Isopropylidendioxy-6β-methoxy-3α,5-cyclo-5α-cholest-22(EZ)-en

Eine Lösung des in b) erhaltenen Produktes (6,46 g) in einem Gemisch von 100 ml Methanol und 50 ml Äthylacetat wird mit 15,2 g 5% Natriumamalgam versetzt. Nach 1 Stunde Rühren bei Raumtemperatur werden weitere 15,2 g, nach 1 Stunde nochmals 15,2 g des Amalgams zugefügt und letztlich noch 3 Stunden gerührt. Die überstehende Lösung wird bei 30°/1467 Pa eingedampft und ihr Rückstand mit Äther und Wasser versetzt. Die ätherische Phase wird mit gesättigter Kochsalz-Lösung gewaschen, über Natriumsulfat getrocknet und bei 30°/1467 Pa eingedampft. Der Rückstand ergibt durch Chromatographie an 150 g Kieselgel mit Hexan/Äther 19 : 1 3,0 g Produkt der Isomerenzusammensetzung E/Z = 3 : 1 ($^1$H-NMR) in Form eines farblosen Harzes.

d) (24R)-24,25-Isopropylidendioxy-6β-methoxy-3α,5-cyclo-5α-cholestan

Eine aus 8 ml äthanolischem Raney-Nickel-Konzentrat und 107 ml Äthanol zubereitete Suspension wird 4 Stunden in Wasserstoffatmosphäre geschüttelt. Eine Lösung von 2,9 g des in c) erhaltenen Produktes in 58 ml Äthanol sowie 1,16 g Natriumhydrogencarbonat werden zugefügt. Dann wird das Gemisch 24 Stunden in Wasserstoffatmosphäre geschüttelt. Die Suspension wird genutscht und das Filtrat bei 40°/1467 Pa eingedampft, wobei 2,78 g Produkt vom Schmelzpunkt 108—110° resultieren. Dieses schmilzt nach Umkristallisation aus Methanol bei 111—112°; $[\alpha]_D^{20} = +43,1°$ (c = 0,7, $CHCl_3$).

e) (24R)-3β,24,25-Trihydroxy-cholest-5-en

Eine Lösung von 2,28 g des in d) erhaltenen Produktes in 23 ml Dioxan wird mit 0,165 g p-Toluolsulfonsäure-monohydrat versetzt, auf 80° erwärmt. Während 10 Minuten werden 40 ml Wasser zugetropft, und die Lösung wird 4 Stunden bei 80° belassen. Nach dem Abkühlen werden 46 ml Wassr zugetropft, das ausgeschiedene Produkt wird abgesaugt, mit Wasser gewaschen und getrocknet: 1,896 g Rohprodukt vom Schmelzpunkt 195—199°. Die Verbindung schmilzt nach Umkristallisation aus Methanol bei 202—204°; $[\alpha]_D^{20} = -9,8°$ (c = 1,0, $CH_3OH$).

f) (24R)-3β,24,25-Triacetoxy-cholest-5-en

Eine Lösung von 1,0 g des in e) erhaltenen Rohproduktes in 10 ml Triäthylamin wird mit 1,8 ml Acetanhydrid und 0,2 g 4-Dimethylaminopyridin versetzt und 3 Stunden unter Rückfluß erwärmt. Die Reaktionslösung wird in Analogie zu b) aufgearbeitet. Der nach der Toluolbehandlung erhaltene kristalline Rückstand liefert durch Chromatographie an 50 g Kieselgel mit Hexan/Äther 9 : 1 und 4 : 1 1,131 g Produkt vom Schmelzpunkt 120—121°. Dieses schmilzt nach Umkristallisation aus Diisopropyläther bei 122—123°; $[\alpha]_D^{20} = -28,1°$ (c = 1,0, $CHCl_3$).

Beispiel 2

a)(22RS,23RS,24R)-23-Hydroxy-24,25-isopropylidendioxy-22-phenylsulfonyl-3β-(tetrahydro-2H-pyran-2-yl)-oxy-cholest-5-en

5,74 g (10,62 mMol) (20S)-20-Methyl-21-phenylsulfonyl-3β-(tetrahydro-2H-pyran-2-yl)-oxy-pregn-5-en ergeben in Analogie zu Beispiel 1a) 7,87 g Produkt.

b) (22RS,23RS,24R)-23-Acetoxy-24,25-isopropylidendioxy-22-phenylsulfonyl-3β-(tetrahydro-2H-pyran-2-yl)-oxy-cholest-5-en

Das in a) erhaltene Produkt ergibt in Analogie zu Beispiel 1b) 7,79 g Acetylierungsprodukt.

c) (24R)-24,25-Isopropylidendioxy-3β-(tetrahydro-2H-pyran-2-yl)-oxy-cholesta-5,22(EZ)-dien

Das in b) gewonnene Produkt wird in einem Gemisch von 168 ml Methanol und 54 ml Äthylacetat gelöst und in Analogie zu Beispiel 1c) mit drei 16,6 g-Portionen 5% Natriumamalgam reduziert und aufgearbeitet. Das Rohprodukt ergibt durch Chromatographie an 150 g Kieselgel mit Hexan/Äther 9 : 1 3,145 g Produkt in Form des E/Z-Gemisches vom Schmelzpunkt 158—160°; $[\alpha]_D^{20} = -53{,}5°$ (c = 1,0, $CHCl_3$). Zweimalige Umkristallisation aus Methanol, enthaltend eine Spur Pyridin, liefert reine 22E-Verbindung vom Schmelzpunkt 172—173°; $[\alpha]_D^{20} = -51{,}2°$ (c = 1,0, $CHCl_3$).

d) (24R)-24,25-Isopropylidendioxy-3β-(tetrahydro-2H-pyran-2-yl)-oxy-cholest-5-en

2,21 g des in c) erhaltenen E/Z-Gemisches ergeben in Analogie zu Beispiel 1d) 2,2 g Produkt vom Schmelzpunkt 112—116°.

e) (24R)-3β,24,25-Trihydroxy-cholest-5-en

Eine Lösung von 1,9 g des in d) gewonnenen Produktes in 19 ml Dioxan wird mit 0,138 g p-Toluolsulfonsäure-monohydrat versetzt, auf 80° erwärmt und nach Zutropfen von 8,3 ml Wasser 3 Stunden bei 80° belassen. Nach dem Abkühlen werden 38 ml Wasser zugetropft, das ausgeschiedene Produkt wird abgesaugt, mit Wasser gewaschen und getrocknet: 1,52 g weißes Pulver.

f) (24R)-3β,24,25-Triacetoxy-cholest-5-en

0,82 g des in e) gewonnenen Produktes ergeben in Analogie zu Beispiel 1f) 0,527 g Produkt vom Schmelzpunkt 118—119°, welches nach Umkristallisation aus Diisopropyläther bei 121—122° schmilzt; $[\alpha]_D^{20} = -26{,}0°$ (c = 0,5, $CHCl_3$).

Beispiel 3

a) (22RS,23RS,24RS)-23-Hydroxy-24,25-isopropylidendioxy-6β-methoxy-22-phenylsulfonyl-3α,5-cyclo-5α-cholestan

5,0 g (10,6 mMol) (20S)-6β-Methoxy-20-methyl-21-phenylsulfonyl-3α,5-cyclo-5α-pregnan ergeben in Analogie zu Beispiel 1a) unter Verwendung von racemischem 2,2,5,5-Tetramethyl-1,3-dioxolan-4-aldehyd 6,94 g Produkt.

b) (22RS,23RS,24RS)-23-Acetoxy-24,25-isopropylidendioxy-6β-methoxy-22-phenylsulfonyl-3α,5-cyclo-5α-cholestan

Das in a) erhaltene Produkt ergibt in Analogie zu Beispiel 1b) 7,0 g Acetylierungsprodukt.

c) (24RS)-24,25-Isopropylidendioxy-6β-methoxy-3α,5-cyclo-5α-cholest-22(EZ)-en

Das in b) gewonnene Acetylierungsprodukt liefert in Analogie zu Beispiel 1c) 3,194 g harzartiges Gemisch der Isomerenzusammensetzung
E(24R) : E(24S) : Z(24R) : Z(24S) = 4 : 4 : 1 : 1 ($^1$H-NMR).

d) (24RS)-24,25-Isopropylidendioxy-6β-methoxy-3α,5-cyclo-5α-cholestan

3,10 g des in c) erhaltenen Gemisches ergibt in Analogie zu Beispiel 1d) 3,09 g Produkt vom Schmelzpunkt 82—84°; $[\alpha]_D^{25} = +44,3°$ (c = 1,0, $CHCl_3$). Eine aus Methanol umkristallisierte Probe schmilzt bei 89—91°; $[\alpha]_D^{20} = +45,4°$ (c = 1,0, $CHCl_3$).

e) (24RS)-3β,24,25-Trihydroxy-cholest-5-en

2,521 g des in d) erhaltenen Produktes liefern in Analogie zu Beispiel 1e) 2,043 g kristallines Material vom Schmelzpunkt 190—196°; $[\alpha]_D^{20} = -30,6°$ (c = 0,5, $CH_3OH$).

f) (24RS)-3β,24,25-Triacetoxy-cholest-5-en

1,2 g des in e) erhaltenen kristallinen Materials liefert in Analogie zu Beispiel 1f) 1,342 g kristallines Produkt vom Schmelzpunkt 117—125°.

g) Chromatographische Auftrennung von (24RS)-3β,24,25-Triacetoxy-cholest-5-en

Eine Lösung von 1,2 g des in f) erhaltenen Produktes in 40 ml Hexan wird auf eine Säule von 100 g neutralem Aluminiumoxid, Akt. III, in Hexan gegeben und nach deren Adsorption mit Hexan/Äther 4 : 1 eluiert. Dabei resultieren vorerst 0,426 g im Dünnschicht-Chromatogramm einheitliches (24R)-3β,24,25-Triacetoxy-cholest-5-en vom Schmelzpunkt 121—123°, das nach Umkristallisation aus Diisopropyläther bei 122—123° schmilzt; $[\alpha]_D^{20} = -28,8°$ (c = 0,5, $CHCl_3$). Nach 0,11 g Mischfraktion werden 0,503 g im Dünnschicht-Chromatogramm einheitliches (24S)-3β,24,25-Triacetoxy-cholest-5-en vom Schmelzpunkt 147—149° eluiert. Die Verbindung schmilzt nach Umkristallisation bei 149—150°; $[\alpha]_D^{20} = -38,8°$ (c = 0,5, $CHCl_3$).

Beispiel 4

a) (22RS,23RS,24R)-1α,3β-Di-(tetrahydro-2H-pyran-2-yl)-oxy-23-hydroxy-24,25-isopropylidendioxy-22-phenylsulfonyl-cholest-5-en

6,80 g (10,62 mMol) (20S)-1α,3β-Di-(tetrahydro-2H-pyran-2-yl)-oxy-20-methyl-21-phenylsulfonyl-pregn-5-en ergeben in Analogie zu Beispiel 1a) 9,26 g Produkt als gelbliches Harz.

b) (22RS,23RS,24R)-23-Acetoxy-1α,3β-di-(tetrahydro-2H-pyran-2-yl)-oxy-24,25-isopropylidendioxy-22-phenylsulfonyl-cholest-5-en

Das in a) erhaltene Produkt ergibt in Analogie zu Beispiel 1b) 8,76 g Acetylierungsprodukt als gelbliches Harz.

c) (24R)-1α,3β,-Di-(tetrahydro-2H-pyran-2-yl)-oxy-24,25-isopropylidendioxy-cholesta-5,22(EZ)-dien

Das in b) gewonnene Produkt wird in Analogie zu Beispiel 1c) mit 5% Natriumamalgam reduziert und aufgearbeitet. Das Rohprodukt liefert durch Chromatographie an 230 g Kieselgel mit Hexan/Äther 9 : 1 und 4 : 1 3,81 g Produkt als E/Z-Gemisch in Form eines gelblichen Harzes.

d) (24R)-1α,3β-Di-(tetrahydro-2H-pyran-2-yl)-oxy-24,25-isopropylidendioxy-cholest-5-en

Das in c) erhaltene Produkt wird in Analogie zu Beispiel 1d) mit Raney-Nickel hydriert. Das Rohprodukt liefert durch Säulenchromatographie an 120 g Kieselgel mit Hexan/Äther 9 : 1 und 4 : 1 3,64 g Produkt in Form eines weißen Harzes.

e) (24R)-1α,3β-Dihydroxy-24,25-isopropylidendioxy-cholest-5-en

Eine Lösung des in d) erhaltenen Produktes in 23 ml Dioxan wird mit 0,16 g p-Toluolsulfonsäure-monohydrat versetzt, auf 80° erwärmt und nach Zutropfen von 9,6 ml Wasser 3 Stunden bei 80° belassen. Nach dem Abkühlen werden 120 ml Wasser zugetropft, das ausgeschiedene Produkt wird abgesaugt, mit Wasser gewaschen und getrocknet: 2,25 g rohes (24R)-1α,3β,24,25-Tetrahydroxy-cholest-5-en vom Schmelzpunkt 195—202°. Das Produkt wird in 68 ml Aceton suspendiert und nach Zutropfen von drei Tropfen 70% Perchlorsäure 30 Minuten bei Raumtemperatur belassen. Die Lösung wird mit 3,4 ml Triäthylamin versetzt und bei 30°/1467 Pa eingedampft. Der Rückstand ergibt durch Chromatographie an 100 g Kieselgel mit Äther und Äthylacetat 1,66 g kristallines Produkt, das nach Umkristallisation aus Methanol bei 174—176° schmilzt, $[\alpha]_D^{20} = -38,4°$ (c=0,5, $CHCl_3$).

f) (24R)-1α,3β,24,25-Tetrahydroxy-cholest-5-en

Eine Lösung von 0,282 g des in e) gewonnenen Produktes in 2,1 ml Dioxan wird mit 0,015 g p-Toluolsulfonsäure-monohydrat versetzt, auf 80° erwärmt und nach Zutropfen von 0,9 ml Wasser 2 Stunden bei 80° belassen. Nach dem Abkühlen werden 10 ml Wasser zugetropft, das ausgeschiedene Produkt wird abgesaugt, mit Wasser gewaschen und getrocknet: 0,219 g weißes Pulver vom Schmelzpunkt 221—222°.

g) (24R)-1α,3β,24,25-Tetraacetoxy-cholest-5-en

Ein Gemisch von 0,21 g des in f) erhaltenen Produktes, 2,1 ml Triäthylamin, 0,38 ml Acetanhydrid und 0,042 g 4-Dimethylaminopyridin wird 3 Stunden unter Rückfluß erwärmt. Die Reaktionslösung wird in Analogie zu Beispiel 1b) aufgearbeitet. Der unter Verwendung von 20 ml Toluol getrocknete Rückstand ergibt durch Chromatographie an 10 g Kieselgel mit Hexan/Äther 4 : 1 0,243 g Produkt als weißes Harz; $[\alpha]_D^{20} = -9,4°$ (c=0,5, $CHCl_3$).

Herstellung der Ausgangsmaterialien

1. (20S)-6β-Methoxy-20-methyl-21-phenylsulfonyl-3α,5-cyclo-5α-pregnan

Eine durch Zugabe von 3,43 g (20S)-21-Jod-6β-methoxy-20-methyl-3α,5-cyclo-5α-pregnan und 2,53 g Natriumbenzolsulfinat zu 23 ml Dimethylformamid hergestellte Mischung wird 20 Minuten bei 80° erwärmt. Die erkaltete Lösung wird in Wasser gegossen und mit Äther extrahiert, der Extrakt mit Wasser gewaschen, über Natriumsulfat getrocknet und bei 30°/1467 Pa eingedampft. Der Rückstand wird an 100 g Kieselgel mit Hexan/Äther 19 : 1 und 9 : 1 chromatographiert. Dabei resultieren als polarer Anteil 3,07 g Produkt, welches nach Umkristallisation aus Äther bei 141—142° schmilzt, $[\alpha]_D^{20} = +55,0°$ (c=0,5, $CHCl_3$).

2. (20S)-20-Methyl-21-phenylsulfonyl-3β-(tetrahydro-2H-pyran-2-yl)-oxy-pregn-5-en

2.1. (20S)-3β-Hydroxy-20-methyl-21-phenylsulfonyl-pregn-5-en

Eine Lösung von 1,0 g (20S)-6β-Methoxy-20-methyl-21-phenylsulfonyl-3α,5-cyclo-5α-pregnan in 10 ml Dioxan wird mit 0,080 g p-Toluolsulfonsäure-monohydrat und 4,4 ml Wasser versetzt und 4 Stunden bei 80° erwärmt. Die gerührte Lösung wird während 5 Minuten mit 10 ml Wasser versetzt und die entstandene Suspension nach dem Abkühlen auf Raumtemperatur genutscht. Der getrocknete Rückstand ergibt durch Chromatographie an 60 g Kieselgel mit Methylenchlorid/Äther 1 : 1 0,897 g Produkt vom Schmelzpunkt 198—199°. Eine aus Methylenchlorid/Äther umkristallisierte Probe schmilzt bei 198—199°; $[\alpha]_D^{20} = -24,1°$ (c=0,5, $CHCl_3$).

2.2. Eine Suspension von 0,597 g des in 2.1. erhaltenen Produktes in 40 ml Benzol wird mit 1,0 ml 3,4-Dihydro-2H-pyran und 0,010 g wasserfreier p-Toluolsulfonsäure versetzt und 30 Minuten bei Raumtemperatur belassen. Nach verdünnen mit Äther wird mit Natriumhydrogencarbonat-Lösung gewaschen, die organische Phase wird über Natriumsulfat getrocknet und bei 30°/1467 Pa eingedampft. Der Rückstand, Smp. 158—161°, ergibt nach Umkristallisation aus Methylenchlorid/Äther, enthaltend 0,1% Triäthylamin, 0,601 g (20S)-20-Methyl-21-phenylsulfonyl-3β-(tetrahydro-2H-pyran-2-yl)-oxy-pregn-5-en vom Schmelzpunkt 160—161°; $[\alpha]_D^{20} = -25,6°$ (c=0,56, $CHCl_3$).

3. (20S)-1$\alpha$,3$\beta$-Di-(tetrahydro-2H-pyran-2-yl)-oxy-20-methyl-21-phenylsulfonyl-pregn-5-en

3.1. (20S)-1$\alpha$,3$\beta$-Diacetoxy-21-jod-20-methyl-pregn-5-ein

Erstes Verfahren

Ein durch Zugabe von 2,34 g (20S)-1$\alpha$,3$\beta$-Diacetoxy-20-methyl-21-p-toluolsulfonyloxy-pregn-5-en und 1,2 g Natriumjodid zu 8 ml Dimethylformamid zubereitetes Gemisch wird 45 Minuten bei 80° erwärmt. Nach dem Abkühlen auf Raumtemperatur wird in Wasser gegossen und mit Hexan extrahiert. Der Extrakt wird mit Wasser gewaschen, über Natriumsulfat getrocknet und bei 30°/1467 Pa eingedampft. Der Rückstand liefert durch Chromatographie an 35 g Kieselgel mit Hexan/Äther 19 : 1 und 9 : 1 1,98 g Produkt, welches nach Umkristallisation aus Pentan bei 136° schmilzt; $[\alpha]_D^{20} = -11{,}0°$ (c = 1,0, $CHCl_3$).

Zweites Verfahren

Eine Lösung von 23,4 g (20S)-1$\alpha$,3$\beta$-Diacetoxy-2-methyl-21-p-toluolsulfonyloxy-pregn-5-en in 260 ml Toluol wird mit 1,04 g Hexadecyl-tri-n-butyl-phosphoniumbromid und einer Lösung von 132 g Natriumjodid in 260 ml Wasser versetzt und 5 Stunden unter Rückfluß erwärmt. Nach dem Abkühlen wird die organische Phase abgetrennt, mit gesättigter Kochsalzlösung gewaschen, über Natriumsulfat getrocknet und bei 30°/1467 Pa eingedampft. Chromatographie des Rückstandes an 270 g Kieselgel mit Hexan/Äther 19 : 1 und 9 : 1 ergibt 21,1 g Produkt vom Schmelzpunkt 135—136°. Durch Umkristallisation aus konz. Pentanlösung werden 16,0 g vom Schmelzpunkt 136° erhalten; $[\alpha]_D^{20} = -11{,}0°$ (c = 1,0, $CHCl_3$).

3.2. (20S)-1$\alpha$,3$\beta$-Diacetoxy-20-methyl-21-phenylsulfonyl-pregn-5-en

21,1 g des in 3.1. gewonnenen Produktes werden in Analogie zu 1. mit 13,1 g Natriumbenzolsulfinat in 120 ml Dimethylformamid umgesetzt, und die Reaktionslösung wird zu Rohprodukt verarbeitet. Dessen Chromatographie an 300 g Kieselgel mit Hexan/Äther 7 : 3 ergibt als polareren Anteil 16,3 g Produkt vom Schmelzpunkt 104° nach Umkristallisation aus Äther/Hexan; $[\alpha]_D^{20} = -7{,}4°$ (c = 1,0, $CHCl_3$).

3.3. (20S)-1$\alpha$,3$\beta$-Dihydroxy-20-methyl-21-phenylsulfonyl-pregn-5-en

Zu einer Suspension von 3,5 g Lithiumaluminiumhydrid in 250 ml Tetrahydrofuran wird unter Rühren bei −20° eine Lösung von 8,15 g des Produktes aus 3.2. in 150 ml Tetrahydrofuran getropft. Nach 2 Stunden Rühren bei −20° werden 400 ml Äthylacetat/Tetrahydrofuran 1 : 1 derart zugetropft, daß die Reaktionstemperatur −8° nicht übersteigt. Das Reaktionsgut wird in ein Gemisch von 250 ml 2M Kaliumnatriumtartrat-Lösung und 150 g Eis gegossen und mit Äthylacetat extrahiert. Der Extrakt wird mit gesättigter Kochsalzlösung gewaschen, über Natriumsulfat getrocknet und bei 30°/1467 Pa eingedampft. Es resultieren 6,9 g Produkt, welches nach Umkristallisation aus Äthylacetat/Methylenchlorid bei 214° schmilzt; $[\alpha]_D^{20} = -12{,}2°$ (c = 0,5, Dioxan).

3.4. Eine Suspension von 1,71 g des Produktes aus 3.3. in 75 ml Benzol wird mit 2,0 ml 3,4-Dihydro-2H-pyran und 0,010 g wasserfreier p-Toluolsulfonsäure versetzt und 2 Stunden bei Raumtemperatur belassen. Die Lösung wird mit gesättigter Natriumhydrogencarbonat-Lösung gewaschen, über Natriumsulfat getrocknet und bei 30°/1467 Pa eingestampft. Chromatographie des Rückstandes an 85 g Kieselgel mit Hexan/Äther 4 : 1 liefert 1,82 g (20S)-1$\alpha$,3$\beta$-Di-(tetrahydro-2H-pyran-2-yl)-oxy-20-methyl-21-phenylsulfonyl-pregn-5-en, das nach Umkristallisation aus Äther/Hexan bei 150—152° schmilzt; $[\alpha]_D^{20} = +12{,}4°$ (c = 1,0, $CHCl_3$).

4. (S)-2,2,5,5-Tetramethyl-1,3-dioxolan-4-aldehyd

4.1. (R)-$\alpha$,$\alpha$,2,2-Tetramethyl-1,3-dioxolan-4-methanol

Zu 120 ml einer 2,95M ätherischen Lösung von Methylmagnesiumbromid wird bei 10—15° unter Rühren eine Lösung von 18,9 g (R)-2,2-Dimethyl-1,3-dioxolan-4-carbonsäure-methylester in 190 ml Äther getropft. Nach 1,45 Stunden Rühren bei Raumtemperatur wird das Gemisch in 1 l gesättigte Ammoniumchlorid-Lösung gegossen, die ätherische Phase abgetrennt und die wäßrige mit Äther extrahiert. Die ätherischen Lösungen werden über Natriumsulfat getrocknet und bei 30°/1467 Pa eingedampft. Chromatographie des Rückstandes an 800 g Kieselgel mit Hexan/Äther 4 : 1 ergibt 15,8 g Produkt als farbloses Öl vom Kp. 125°/1600 Pa (Kugelrohr); $[\alpha]_D^{20} = +17{,}2°$ (c = 2,0, $CHCl_3$).

4.2. (R)-3-Methyl-1,2,3-butantriol

Eine Lösung von 8,9 g Produkt aus 4.1. in 180 ml 90% wäßrigem Tetrahydrofuran wird mit 0,52 g p-Toluolsulfonsäure-monohydrat versetzt und 3 Stunden bei 70° erwärmt. Nach Zugabe von 4 ml Triäthylamin wird bei 30°/1467 Pa eingedampft, der Rückstand in Benzol aufgenommen und abermals bei 1467 Pa zur Trockne eingedampft. Chromatographie des Rückstandes an 90 g Kieselgel mit Äthylacetat und Äthylacetat/Tetrahydrofuran 1 : 1 ergibt 5,98 g Produkt als farbloses Öl vom Kp. 125°/267 Pa (Kugelrohr); $[\alpha]_D^{20} = +23{,}5°$ (c = 1,0, $CH_3OH$).

4.3. (R)-(2,3-Dihydroxy-3-methylbutyl)-pivalat

Eine Lösung von 2,87 g Produkt aus 4.2. in 15 ml Pyridin wird bei 0° mit 2,93 ml Pivalinsäurechlorid versetzt und das Gemisch 15 Minuten bei 0° sowie 1,5 Stunden bei Raumtemperatur gerührt. Die Suspension wird mit 50 ml Äther verdünnt, genutscht und das Filtrat bei 30°/1467 Pa eingedampft. Nach Entfernung restlicher Mengen Pyridin durch Lösen in o-Xylol und Eindampfen bei 50°/1467 Pa wird der Rückstand an 140 g Kieselgel mit Hexan/Äther 1 : 1 chromatographiert. Dabei resultieren 4,04 g öliges Produkt mit $[\alpha]_D^{20} = +18{,}2°$ (c = 1,0, $CHCl_3$).

4.4. (R)-(2,2,5,5-Tetramethyl-1,3-dioxolan-4-yl)-methyl-pivalat

Eine Lösung von 3,81 g des in 4.3. erhaltenen Produktes in 85 ml Aceton wird mit 0,28 g wasserfreier p-Toluolsulfonsäure versetzt und 3 Stunden bei Raumtemperatur belassen. Nach Zufügen von 1 ml Triäthylamin wird bei 30°/1467 Pa eingedampft und der Rückstand an 100 g Kieselgel mit Hexan/Äther 9 : 1 chromatographiert. Dabei werden 4,1 g Produkt als farbloses Öl vom Kp. 115°/1600 Pa (Kugelrohr) erhalten; $[\alpha]_D^{20} = -5{,}25°$ (c = 0,8, $CHCl_3$).

4.5. (R)-2,2,5,5-Tetramethyl-1,3-dioxolan-4-methanol

4.5.1. Aus (R)-$\alpha$,$\alpha$,2,2-Tetramethyl-1,3-dioxolan-4-methanol

Eine Lösung von 3,5 g Produkt aus 4.1. in 60 ml Aceton wird mit 0,030 g wasserfreier p-Toluolsulfonsäure versetzt und 5 Tage bei Raumtemperatur belassen. Nach Zugabe von 1 ml Triäthylamin wird bei 30°/1467 Pa eingedampft und der Rückstand an 210 g neutralem Aluminiumoxid, Akt. III, mit Methylenchlorid chromatographiert. Dabei wird neben 1,81 g Edukt als polare Fraktion 0,90 g Produkt als farbloses Öl vom Kp. 125°/1600 Pa erhalten; $[\alpha]_D^{20} = -13{,}1°$ (c = 0,7, $CHCl_3$).

4.5.2. Aus (R)-(2,2,5,5-Tetramethyl-1,3-dioxolan-4-yl)-methyl-pivalat

Eine Lösung von 3,88 g Produkt aus 4.4. in 65 ml 0,5N methanolischer Natronlauge wird 4 Stunden bei Raumtemperatur belassen und anschließend bei 30°/1467 Pa eingedampft. Chromatographie des Rückstandes an 100 g Kieselgel mit Pentan/Äther 9 : 1 ergibt 2,36 g Produkt als farbloses Öl; $[\alpha]_D^{25} = -15{,}0°$ (c = 1,0, $CHCl_3$).

4.6. (S)-2,2,5,5-Tetramethyl-1,3-dioxolan-4-aldehyd

Zu einem durch Zugabe von 12,5 g wasserfreiem Calciumsulfat und 4,16 g Pyridiniumchlorochromat zu 25 ml Methylenchlorid hergestelltem Gemisch wird bei Raumtemperatur unter Rühren eine Lösung von 2,0 g des Produktes aus 4.5.2. in 4 ml Methylenchlorid gefügt. Nach 70 Minuten Rühren bei Raumtemperatur wird das Gemisch auf eine in Methylenchlorid bereitete Säule von 40 g Kieselgel gegeben und mit Methylenchlorid gewaschen. Die nach Dünnschicht-Chromatographie einheitlichen Eluate ergeben nach Vereinigung, Abdampfen des Lösungsmittels bei 50° und Destillation des Rückstandes bei 105°/1600 Pa (Kugelrohr) 1,0 g Produkt als farbloses Öl mit $[\alpha]_D^{20} = -138{,}6°$ (c = 1,0, Hexan).

5. (RS)-2,2,5,5-Tetramethyl-1,3-dioxolan-4-aldehyd

5.1. (RS)-(2,3)-Dihydroxy-3-methylbutyl)-pivalat

Aus 3,0 g (RS)-3-Methyl-1,2,3-butantriol wird in Analogie zu 4.3. 4,42 g Produkt erhalten, welches nach Umkristallisation aus Äther bei 75–76° schmilzt.

5.2. (RS)-(2,2,5,5-Tetramethyl-1,3-dioxolan-4-yl)-methyl-pivalat

Aus 1,34 g Produkt aus 5.1. wird in Analogie zu 4.4. 1,46 g Produkt erhalten, das nach Umkristallisation aus Hexan bei 62—63° schmilzt.

5.3. (RS)-1-Benzyloxy-3-methyl-2,3-butandiol

Eine Lösung von 0,460 g Natrium in 10 ml Äthanol wird mit 2,4 g (RS)-3-Methyl-1,2,3-butantriol versetzt. 20 ml Toluol werden zugefügt, und unter gleichzeitigem Zutropfen von 60 ml Toluol wird langsam Lösungsmittel abdestilliert, bis das Destillat 75 ml beträgt. Die verbliebene Suspension wird genutscht, der Rückstand mit Äther gewaschen und bei Raumtemperatur/1467 Pa getrocknet. Das Salz wird in 10 ml Tetrahydrofuran suspendiert, dann werden 0,740 g Tetrabutylammoniumjodid und 4,0 ml Benzylbromid zugegeben, und es wird 3 Tage bei Raumtemperatur gerührt. Das Gemisch wird mit Benzol verdünnt und bei 30°/1467 Pa konzentriert. Der Rückstand wird an 30 g Kieselgel mit Hexan/Äther 4 : 1 chromatographiert. Dabei resultieren neben 0,616 g (RS)-1,2-Dibenzyloxy-3-methyl-3-butanol als polarere Fraktion 1,15 g an gewünschtem Produkt als farbloses Öl vom Kp. 150°/267 Pa (Kugelrohr).

5.4. (RS)-5-Benzyloxymethyl-2,2,4,4-tetramethyl-1,3-dioxolan

Eine Lösung von 1,15 g des Produktes aus 5.3. in 100 ml Aceton wird mit 0,050 g wasserfreier p-Toluolsulfonsäure versetzt und 16 Stunden bei Raumtemperatur belassen. Nach Zugabe von 1 ml Triäthylamin wird bei 30°/1467 Pa eingedampft. Chromatographie des Rückstandes an 10 g Kieselgel mit Hexan/Äther 4 : 1 ergibt 1,34 g Produkt als Öl vom Kp. 150°/267 Pa (Kugelrohr).

5.5. (RS)-2,2,5,5-Tetramethyl-1,3-dioxolan-4-methanol

5.5.1. Aus (RS)-(2,2,5,5-Tetramethyl-1,3-dioxolan-4-yl)-methyl-pivalat

Aus 0,642 g Produkt aus 5.2. wird in Analogie zu 4.5.2. 0,382 g Produkt als Öl vom Kp. 130°/1600 Pa (Kugelrohr) erhalten.

5.5.2. Aus (RS)-5-Benzyloxymethyl-2,2,4,4-tetramethyl-1,3-dioxolan

Zu einem vorhydrierten Gemisch aus 0,50 g 5% Palladiumkohle, 0,10 g Natriumhydrogencarbonat und 10 ml Äthylacetat werden 0,51 g Produkt aus 5.4. gefügt, und es wird bei Raumtemperatur in Wasserstoffatmosphäre geschüttelt. Nach beendeter Gasaufnahme wird vom Katalysator abfiltriert, und das Filtrat wird bei 30°/1467 Pa eingedampft. Der Rückstand ergibt durch Destillation bei 130°/1600 Pa (Kugelrohr) 0,30 g Produkt als farbloses Öl.

5.5.3. Aus (RS)-3-Methyl-1,2,3-butantriol

Eine Lösung von 1,2 g (RS)-3-Methyl-1,2,3-butantriol in 20 ml Aceton wird mit 0,010 g wasserfreier p-Toluolsulfonsäure versetzt und 5 Tage bei Raumtemperatur belassen. Nach Zugabe von 0,3 ml Triäthylamin wird bei 30°/1467 Pa eingedampft, und der Rückstand wird bei 130°/1467 Pa destilliert. Das Destillat, 1,55 g, ergibt bei der Chromatographie an 90 g neutralem Aluminiumoxid, Akt. III, mit Methylenchlorid als unpolarere Fraktion 0,81 g (RS)-α,α,2,2-Tetramethyl-1,3-dioxolan-4-methanol und als polarere 0,52 g an gewünschtem Produkt.
Bei einem in Analogie mit 12,7 g Edukt ausgeführten Ansatz wird das Rohprodukt durch fraktionierte Destillation aufgetrennt. Dabei resultieren 7,1 g (RS)-α,α,2,2-Tetramethyl-1,3-dioxolan-4-methanol vom Kp. 33—34°/267 Pa, 0,5 g Mischfraktion (Kp. 34—38°/267 Pa) und als Rückstand 5,6 g an gewünschtem Produkt.

5.6. (RS)-2,2,5,5-Tetramethyl-1,3-dioxolan-4-aldehyd

4,0 g des Produktes aus 5.5.3. ergeben in Analogie zu 4.6. 2,38 g Produkt als farbloses Öl vom Kp. 105°/1600 Pa.

**Patentansprüche**

1. Verfahren zur Herstellung von Cholesterinderivaten der Formel

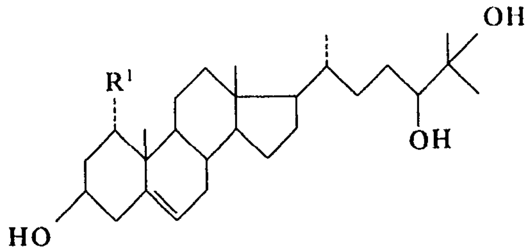

(I)

worin $R^1$ Wasserstoff oder Hydroxy ist, dadurch gekennzeichnet, daß man

a) ein Pregnanderivat der Formel

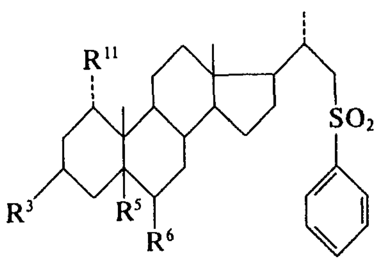

(II)

worin $R^{11}$ Wasserstoff, $R^3$ und $R^5$ zusammen eine $3\alpha$,5-Bindung und $R^6$ $C_{1-4}$-Alkoxy sind, oder $R^{11}$ Wasserstoff oder eine leicht spaltbare verätherte Hydroxygruppe, $R^3$ eine leicht spaltbare verätherte Hydroxygruppe und $R^5$ und $R^6$ zusammen eine C—C-Bindung sind, mit einer Verbindung der Formel

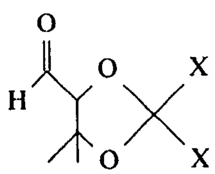

(III)

worin X Wasserstoff oder Methyl ist, umsetzt,

b) aus der erhaltenen Verbindung der Formel

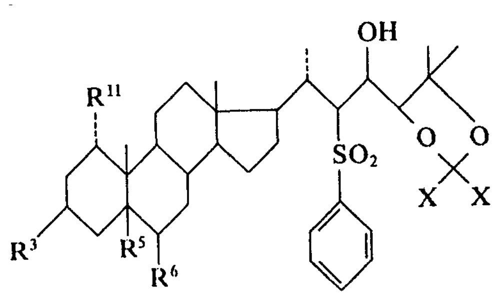

(IV)

worin X, $R^{11}$, $R^3$, $R^5$ und $R^6$ die obige Bedeutung haben, die $\beta$-Hydroxysulfongruppierung reduktiv eliminiert,

c) die erhaltene Verbindung der Formel

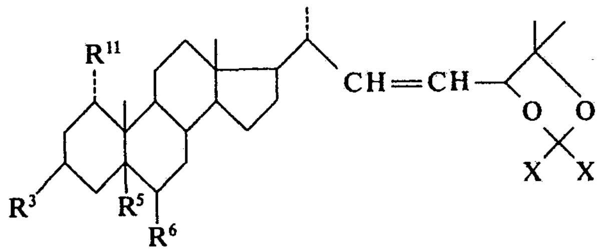

(V)

worin X, $R^{11}$, $R^3$, $R^5$ und $R^6$ die obige Bedeutung haben, katalytisch hydriert,

d) die erhaltene Verbindung der Formel

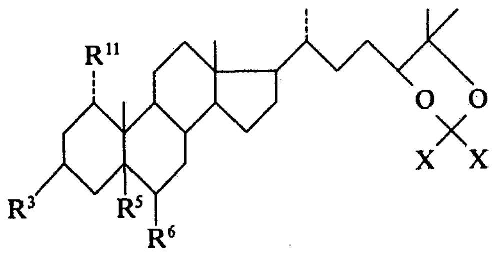

(VI)

worin X, $R^{11}$, $R^3$, $R^5$ und $R^6$ die obige Bedeutung haben, zu einer Verbindung der Formel I hydrolysiert und

e) erwünschtenfalls ein erhaltenes Diastereomerengemisch von Verbindungen der Formel I auftrennt.

2. Verfahren nach Anspruch 1, dadurch gekennzeichnet, daß man als Ausgangsmaterial der Formel III ein solches verwendet, worin X Methyl ist, insbesondere (S)-2,2,5,5-Tetramethyl-1,3-dioxolan-4-aldehyd.

3. Verfahren nach Anspruch 1 oder 2, dadurch gekennzeichnet, daß man zur Durchführung der Stufe b) zunächst die Hydroxygruppe in 23-Stellung des Cholestanols der Formel IV n-$C_{1-4}$-alkanoyliert und die $\beta$-n-$C_{1-4}$-Alkanoyloxysulfongruppierung aus dem erhaltenen Ester reduktiv eliminiert.

4. Verfahren nach Anspruch 1, dadurch gekennzeichnet, daß man zur Durchführung der Stufe a) zunächst die Verbindung der Formel II mit einem $C_{1-4}$-Alkylmagnesiumhalogenid oder $C_{1-4}$-Alkyllithium in einem inerten Lösungsmittel zum Magnesium- bzw. Lithiumsalz und anschließend letzteres mit dem Aldehyd der Formel III umsetzt.

5. Ein Cholest-22-enderivat der Formel

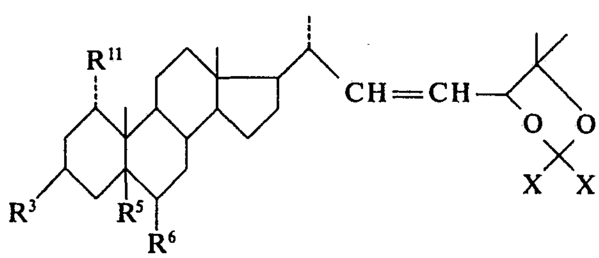

(V)

worin $R^{11}$ Wasserstoff, $R^3$ und $R^5$ zusammen eine $3\alpha$,5-Bindung und $R^6$ $C_{1-4}$-Alkoxy sind, oder $R^{11}$ Wasserstoff oder eine leicht spaltbare verätherte Hydroxygruppe, $R^3$ eine leicht spaltbare verätherte Hydroxygruppe und $R^5$ und $R^6$ zusammen eine C—C-Bindung sind, und X Wasserstoff oder Methyl ist.

6. Eine Verbindung gemäß Anspruch 5 aus der Gruppe

(24R)-24,25-Isopropylidendioxy-$6\beta$-methoxy-$3\alpha$,5-cyclo-$5\alpha$-cholest-22(EZ)-en,
(24RS)-24,25-Isopropylidendioxy-$6\beta$-methoxy-$3\alpha$,5-cyclo-$5\alpha$-cholest-22(EZ)-en,
(24R)-24,25-Isopropylidendioxy-$3\beta$-(tetrahydro-2H-pyran-2-yl)-oxy-cholesta-5,22(EZ)-dien,
(24R)-$1\alpha$,$3\beta$-Di-(tetrahydro-2H-pyran-2-yl)-oxy-24,25-isopropylidendioxy-cholesta-5,22(EZ)-dien.

7. Ein Pregnanderivat der Formel

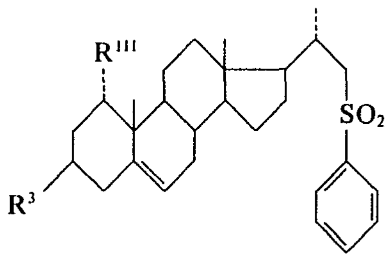

(IIc)

worin $R^{111}$ und $R^3$ leicht spaltbare verätherte Hydroxygruppen sind.

8. Das (20S)-1α,3β-Di-(tetrahydro-2H-pyran-2-yl)-oxy-20-methyl-21-phenylsulfonyl-pregn-5-en.

**Claims**

1. A process for the manufacture of cholesterol derivatives of the formula

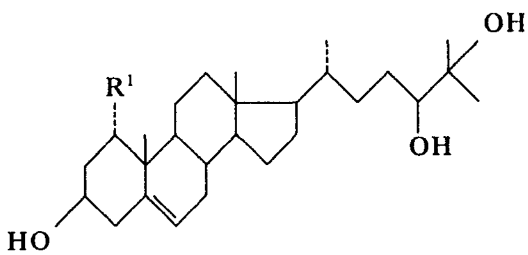

(I)

wherein $R^1$ is hydrogen or hydroxy,
characterized by

a) reacting a pregnane derivative of the formula

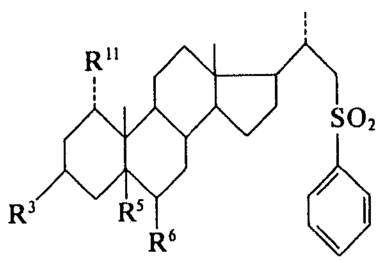

(II)

wherein $R^{11}$ is hydrogen, $R^3$ and $R^5$ together are a 3α,5-bond and $R^6$ is $C_{1-4}$-alkoxy, or $R^{11}$ is hydrogen or a readily cleavable etherified hydroxy group, $R^3$ is a readily cleavable etherified hydroxy group and $R^5$ and $R^6$ together are a C—C-bond, with a compound of the formula

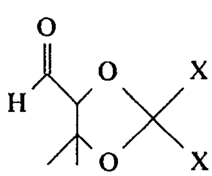

(III)

wherein X is hydrogen or methyl,

b) reductively eliminating the $\beta$-hydroxysulphone grouping from the resulting compound of the formula

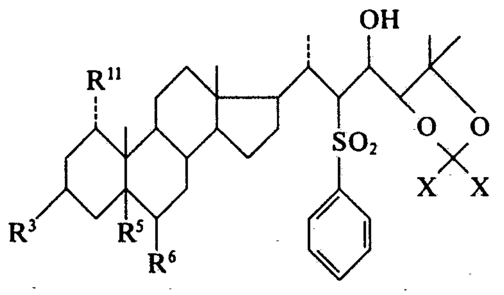

(IV)

wherein X, $R^{11}$, $R^3$, $R^5$ and $R^6$ have the above significance,

c) catalytically hydrogenating the resulting compound of the formula

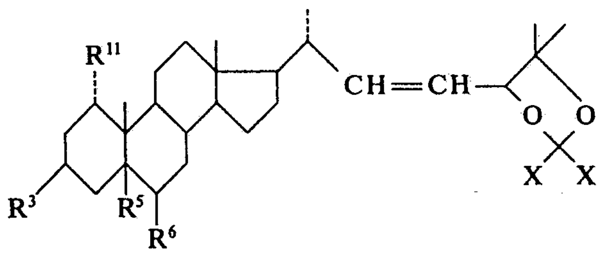

(V)

wherein X, $R^{11}$, $R^3$, $R^5$ and $R^6$ have the above significance,

d) hydrolyzing the resulting compound of the formula

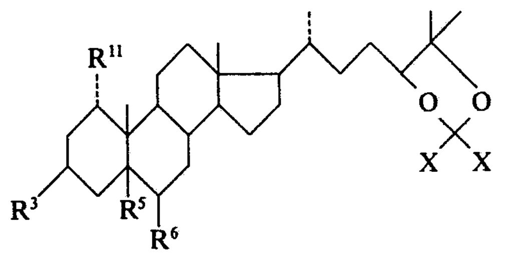

(VI)

wherein X, $R^{11}$, $R^3$, $R^5$ and $R^6$ have the above significance, to a compound of formula I and

e) if desired, separating a diastereomeric mixture of compounds of formula I obtained.

2. A process according to claim 1, characterized in that the starting material of formula III used is one in which X is methyl, especially (S)-2,2,5,5-tetramethyl-1,3-dioxolan-4-aldehyde.

3. A process according to claim 1 or 2, characterized in that step b) is carried out by firstly n-$C_{1-4}$-alkanoylating the hydroxy group in the 23-position of the cholestanol of formula IV and reductively eliminating the $\beta$-n-$C_{1-4}$-alkanoyloxysulphone grouping from the ester obtained.

4. A process according to claim 1, characterized in that step a) is carried out by firstly reacting the compound of formula II with a $C_{1-4}$-alkylmagnesium halide or $C_{1-4}$-alkyl lithium in an inert solvent to give the magnesium or lithium salt and subsequently reacting the latter with the aldehyde of formula III.

5. A cholest-22-ene derivative of the formula

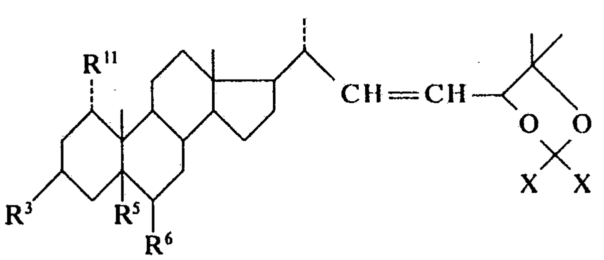

(V)

wherein $R^{11}$ is hydrogen, $R^3$ and $R^5$ together are a $3\alpha$,5-bond and $R^6$ is $C_{1-4}$-alkoxy, or $R^{11}$ is hydrogen or a readily cleavable etherified hydroxy group, $R^3$ is a readily cleavable etherified hydroxy group and $R^5$ and $R^6$ together are a C—C bond, and X is hydrogen or methyl.

6. A compound in accordance with claim 5 from the group:

(24R)-24,25-isopropylidenedioxy-6β-methoxy-3α,5-cyclo-5α-cholest-22(EZ)-ene,
(24RS)-24,25-isopropylidenedioxy-6β-methoxy-3α,5-cyclo-5α-cholest-22(EZ)-ene,
(24R)-24,25-isopropylidenedioxy-3β-(tetrahydro-2H-pyran-2-yl)-oxy-cholesta-5,22(EZ)-diene,
(24R)-1α,3β-di-(tetrahydro-2H-pyran-2-yl)-oxy-24,25-isopropylidenedioxy-cholesta-5,22(EZ)-diene.

7. A pregnane derivative of the formula

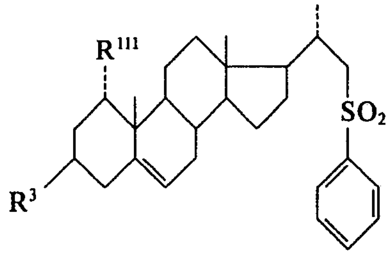

(IIc)

wherein $R^{111}$ and $R^3$ are readily cleavable etherified hydroxy groups.

8. (20S)-1α,3β-Di-(tetrahydro-2H-pyran-2-yl)-oxy-20-methyl-21-phenylsulphonyl-pregn-5-ene.

## Revendications

1. Procédé de préparation de dérives de cholestérol de formule

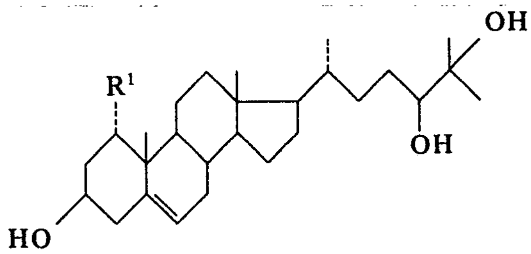

(I)

où $R^1$ est un hydrogène ou un hydroxy,
caractérisé en ce que

a) on fait réagir un dérivé prégnane de formule

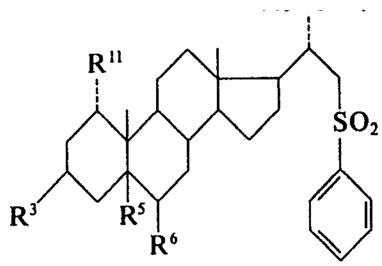

(II)

où $R^{11}$ représente un hydrogène, $R^3$ et $R^5$ représentent ensemble une liaison $3\alpha$,5 et $R^6$ représente un alcoxy en $C_1$ à $C_4$,
ou bien où $R^{11}$ représente un hydrogène ou un groupe hydroxy éthérifié facilement séparable, $R^3$ représente un groupe hydroxy éthérifié facilement séparable et $R^5$ et $R^6$ représentent ensemble une liaison C—C,

avec un composé de formule

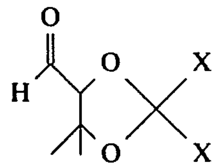

(III)

où X est un hydrogène ou un méthyle,

b) on élimine par réduction le groupement $\beta$-hydroxysulfone dans un composé obtenu de formule

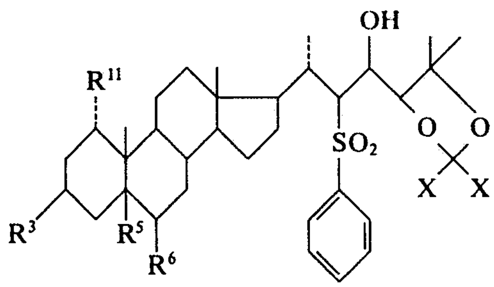

(IV)

où X, $R^{11}$, $R^3$, $R^5$ et $R^6$ ont la signification donnée ci-dessus,

c) on hydrogène de façon catalytique le composé obtenu de formule

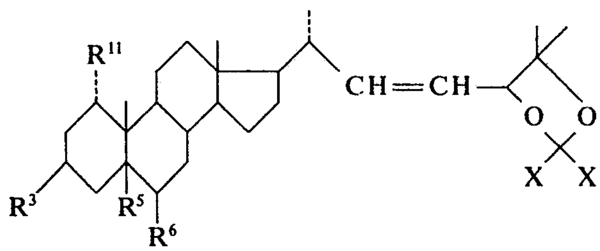

(V)

où X, $R^{11}$, $R^3$, $R^5$ et $R^6$ ont la signification donnée ci-dessus,

d) on hydrolyse le composé obtenu de formule

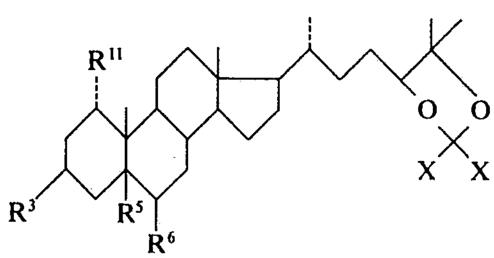

(VI)

où X, $R^{11}$, $R^3$, $R^5$ et $R^6$ ont la signification donnée ci-dessus, pour donner un composé de formule I et

e) si on le désire on sépare un mélange de diastéréomères de composés de formule I obtenu.

2. Procédé selon la revendication 1, caractérisé en ce qu'on utilise comme produit de départ de formule III un corps où X est un méthyle, en particulier le (S)-2,2,5,5-tétraméthyl-1,3-dioxolan-4-aldéhyde.

3. Procédé selon les revendications 1 et 2, caractérisé en ce que pour réaliser l'étape b) on commence par traiter avec un n-alcanoyle en $C_1$ à $C_4$ le groupe hydroxy en position 23 du cholestanol de formule IV et on élimine par réduction de l'ester obtenu de groupement $\beta$-n-alcanoyloxy en $C_1$ à $C_4$-sulfone.

4. Procédé selon la revendication 1, caractérisé en ce que pour réaliser l'étape a) on fait réagir tout d'abord le composé de formule II avec un halogénure d'alcoyle en $C_1$ à $C_4$ magnésium ou un alcoyle en $C_1$ à $C_4$-lithium dans un solvant inerte pour donner le sel de magnésium ou de lithium puis on afait réagir ce dernier avec l'aldéhyde de formule III.

5. Dérivé de cholest-22-ène de formule

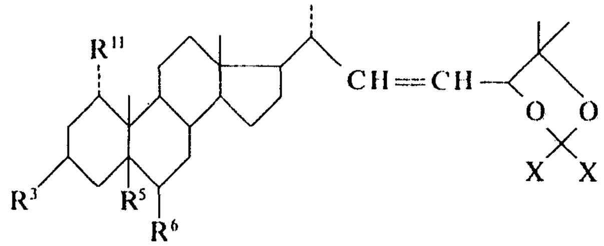

(V)

où $R^{11}$ est un hydrogène, $R^3$ et $R^5$ ensemble une liaison 3α,5 et $R^6$ un alcoxy en $C_1$ à $C_4$, ou bien où $R^{11}$ est un hydrogène ou un groupe hydroxy éthérifié facilement séparable, $R^3$ est un groupe hydroxy éthérifié facilement séparable, et $R^5$ et $R^6$ ensemble une liaison C—C, et X un hydrogène ou un méthyle.

6. Composé selon la revendication 5:

(24R)-24,25-Isopropylidènedioxy-6β-méthoxy-3α,5-cyclo-5α-cholest-22(EZ)-ène,
(24RS)-24,25-Isopropylidènedioxy-6β-méthoxy-3α,5-cyclo-5α-cholest-22(EZ)-ène,
(24R)-24,25-Isopropylidènedioxy-3β-(tétrahydro-2H-pyran-2-yl)-oxy-cholesta-5,22(EZ)-diène,
(24R)-1α,3β-Di-(tétrahydro-2H-pyran-2-yl)-oxy-24,25-isopropylidènedioxy-cholesta-5,22(EZ)-diène.

7. Dérivé prégnane de formule

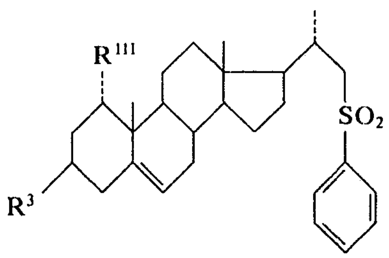

(IIc)

où $R^{111}$ et $R^3$ représentent des groupes hydroxy éthérifiés facilement séparables.

8. (20S)-1α,3β-Di-(tétrahydro-2H-pyran-2-yl)-oxy-20-méthyl-21-phénylsulfonyl-pregn-5-ène.